(19) Europäisches Patentamt
European Patent Office
Office européen des brevets

(11) EP 2 065 073 B1

(12) FASCICULE DE BREVET EUROPEEN

(45) Date de publication et mention de la délivrance du brevet:
**19.08.2015 Bulletin 2015/34**

(51) Int Cl.:
***A61Q 5/10*** *(2006.01)* ***A61K 8/73*** *(2006.01)*
***A61K 8/34*** *(2006.01)*

(21) Numéro de dépôt: **08168645.3**

(22) Date de dépôt: **07.11.2008**

(54) **Composition de teinture d'oxydation des fibres kératiniques comprenant une cellulose à substituants hydrophobes, un colorant d'oxydation et un alcool gras à chaine longue**

Zusammensetzung zum Oxidationsfärben von Keratinfasern enthaltend eine Cellulose mit hydrophobischen Gruppe, eine Oxidationsfarbmittel und ein fettes Alkohol

Composition for the oxidation dyeing of keratin fibres comprising a cellulose with hydrophobic substituents, an oxidation dye and a long-chain fatty alcohol

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **09.11.2007 FR 0758930**

(43) Date de publication de la demande:
**03.06.2009 Bulletin 2009/23**

(73) Titulaire: **L'Oréal**
**75008 Paris (FR)**

(72) Inventeur: **Audousset, Marie-Pascale**
**92600 Asnieres (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 1 707 190** **WO-A-98/03150**
**FR-A- 2 803 197**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 065 073 B1

## Description

La présente demande a pour objet une composition de teinture d'oxydation des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences.

Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (i.e. abîmées) de sa pointe à sa racine.

Par ailleurs, les compositions obtenues doivent, en outre, présenter de bonnes propriétés rhéologiques, tout en conservant de bonnes propriétés de coloration. En particulier, ces compositions ne doivent pas couler sur le visage ou en dehors des zones que l'on se propose de teindre, lors de leur application, notamment après mélange avec un agent oxydant.

Il est déjà connu de la demande WO 98/03150 d'améliorer la puissance de la coloration en associant une base d'oxydation para-phénylènediamine et au moins un polymère amphiphile non ionique du type hydroxycellulose modifiée par un groupement hydrophobe. Le document EP-A-1707190 décrit une composition colorante comprenant une cellulose non ionique modifiée par un groupement hydrophobe. Le document FR-A-2803197 décrit une composition pour la coloration des cheveux qui contient un polymère épaississant à chaîne grasse et un alcool gras à plus de 20 atomes de carbone.

Cependant, ces compositions ne satisfont pas pleinement les exigences précitées et peuvent être améliorées, notamment en termes de propriétés tinctoriales, en particulier au niveau de la sélectivité et de la puissance de coloration. Le but de la présente invention est l'obtention de compositions de coloration capillaire stables, notamment sous forme de crèmes, faciles à préparer et à appliquer, pouvant contenir des concentrations élevées de colorants sous forme de sels, ayant de bonnes qualités rhéologiques et conduisant à des colorations intenses, peu sélectives et résistantes aux diverses agressions que peuvent subir les fibres kératiniques.

Ce but est atteint par la présente invention qui a pour objet une composition tinctoriale pour fibres kératiniques, et en particulier pour fibres kératiniques humaines telles que les cheveux, comprenant, dans un milieu approprié pour la teinture :

(A) un ou plusieurs dérivé(s) non ionique(s) de cellulose comprenant un ou plusieurs substituant(s) hydrophobe(s) comprenant de 8 à 30 atomes de carbone en quantité comprise entre 0,1 et 1 % en poids par rapport au poids total de la composition ,
(B) un ou plusieurs colorants d'oxydation, et
(C) un ou plusieurs alcools gras comprenant au moins 20 atomes de carbone.

Les compositions tinctoriales selon l'invention présentent notamment les propriétés suivantes :

- elles permettent d'obtenir des compositions de viscosité correspondant à une crème qui sont stables dans le temps,
- elles se distinguent par une facilité de mélange avec la composition oxydante,
- elles se distinguent par les qualités rhéologiques des crèmes obtenues (bonne viscosité de crème en mélange),
- elles sont faciles à appliquer après mélange avec la composition oxydante au moment de la mise en oeuvre de la coloration (qualités d'usage sur tête).

En outre, les compositions selon l'invention permettent l'obtention de compositions capables de conduire à des colorations aux nuances variées, chromatiques, puissantes, esthétiques, peu sélectives, uniformes sur l'ensemble

des fibres kératiniques et en particulier des fibres kératiniques humaines telles que les cheveux, et résistant bien aux diverses agressions que peuvent subir les fibres.

Un autre objet de la présente invention consiste en un procédé de teinture des fibres kératiniques dans lequel la composition cosmétique selon l'invention est mise en oeuvre.

Un troisième objet de l'invention concerne l'utilisation de cette composition cosmétique pour la teinture des fibres kératiniques, et en particulier des fibres kératiniques humaines, telles que les cheveux.

D'autres caractéristiques, aspects, objets et avantages de la présente invention apparaîtront encore plus clairement à la lecture de la description et des exemples qui suivent.

A moins d'une indication différente, les bornes des gammes de valeurs qui sont données dans le cadre de la présente invention sont incluses dans ces gammes.

Par « dérivé(s) de cellulose », on entend un (ou des) composé(s) comportant au moins un motif cellobiose de structure suivante :

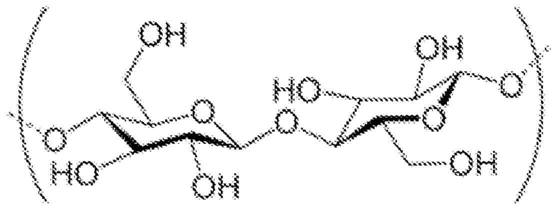

dans laquelle, un ou plusieurs groupe(s) hydroxyle peut (ou peuvent) être substitué(s).

Le ou les dérivés non ioniques de cellulose à substituant(s) hydrophobe(s) (A) conformes à la présente invention, sont des polymères amphiphiles présentant un caractère associatif. En effet, ils comprennent des motifs hydrophiles et des motifs hydrophobes et sont capables d'interagir et de s'associer entre eux ou à d'autres molécules, de manière réversible, en particulier, grâce à la présence de leurs chaînes hydrophobes.

De préférence, le dérivé de cellulose de l'invention est un éther de cellulose comprenant un ou plusieurs substituant(s) hydrophobe(s) comprenant de 8 à 30 atomes de carbone.

Le ou les dérivés non ioniques de cellulose à substituant(s) hydrophobe(s) conformes à la présente invention, sont préparés généralement à partir d'éthers non-ioniques de cellulose hydrosolubles, dont on substitue tout ou partie des fonctions hydroxyle réactives par une ou plusieurs chaîne(s) hydrophobe(s) comprenant de 8 à 30 atomes de carbone, de préférence de 10 à 22 atomes de carbone, et mieux encore 16 atomes de carbone. Les étapes de réactions en jeu dans la préparation des dérivés de cellulose de l'invention sont connues de l'homme du métier.

Les éthers non-ioniques de cellulose choisis pour préparer les dérivés non-ioniques de cellulose à substituant(s) hydrophobe(s) selon l'invention, ont de préférence un degré de substitution non-ionique, par exemple en groupement(s) méthyle, hydroxyéthyle ou hydroxypropyle, suffisant pour être hydrosolubles, c'est-à-dire former une solution sensiblement limpide lorsqu'ils sont dissous dans l'eau à 25 °C à la concentration de 1 % en poids.

Les éthers non ioniques de cellulose choisis pour préparer les dérivés non ioniques de cellulose à substituant(s) hydrophobe(s) selon l'invention, possèdent préférentiellement une masse molaire moyenne en nombre relativement faible, inférieure à 800 000 g/mole, de préférence allant de 50 000 et 700 000 g/mole et de manière plus préférée allant de 200 000 à 600 000 g/mole.

De préférence, le dérivé de cellulose de l'invention est une hydroxyéthylcellulose comprenant un ou plusieurs substituant(s) hydrophobe(s) comprenant de 8 à 30 atomes de carbone.

Les dérivés non-ioniques de cellulose utilisés selon l'invention sont substitués, par une ou plusieurs chaîne(s) hydrocarbonée(s) en $C_8$-$C_{30}$ linéaires, ramifiées ou cycliques, saturées ou insaturées, aliphatiques ou aromatiques, pouvant être rattachées au substrat éther de cellulose par une liaison éther, ester, ou uréthane, de préférence éther.

Selon un mode de réalisation, le ou les substituants hydrophobes utilisés comme substituants des dérivés non-ioniques de cellulose selon la présente invention sont des groupes alkyle, arylalkyle ou alkylaryle en $C_8$-$C_{30}$, de préférence en $C_{10}$-$C_{22}$.

De préférence, le ou les substituants hydrophobes selon la présente invention sont des chaînes alkyles saturées.

Selon un mode de réalisation préféré, le ou les substituants hydrophobes selon la présente invention sont des groupements cétyle.

Les dérivés non-ioniques de cellulose à substituant(s) hydrophobe(s) selon l'invention, présentent une viscosité de préférence comprise entre 100 et 100 000 mPa.s, et de préférence entre 200 et 20 000 mPa.s, mesurée à 25°C dans une solution à 1 % en poids de polymère dans l'eau, cette viscosité étant déterminée de manière conventionnelle à l'aide d'un viscosimètre de type Brookfield LVT à 6 tours par minute avec le mobile n° 3.

Le degré de substitution hydrophobe des dérivés non-ioniques de cellulose hydrophiles utilisés selon l'invention, va préférentiellement de 0,1 à 10% en poids, plus préférentiellement de 0,1 à 1% en poids et de manière particulièrement

préférée de 0,4 à 0,8% en poids, du poids total du polymère.

Parmi les dérivés non-ioniques de cellulose à substituant(s) hydrophobe(s) utilisables dans les compositions de l'invention, on peut citer, de préférence, les cétyl hydroxyéthylcelluloses commercialisées sous les dénominations Natrosol Plus Grade 330 CS et Polysurf 67 CS (INCI : Cetyl Hydroxyethylcellulose) par la société Aqualon/Hercules.

La ou les colorants d'oxydation utiles dans la présente invention peuvent être choisis parmi les bases d'oxydation et les coupleurs.

Le (ou les) colorant(s) d'oxydation (C) utilisable(s) selon l'invention est (ou sont) de préférence choisi(s) parmi les bases d'oxydation, les coupleurs d'oxydation, et leurs sels d'addition.

A titre d'exemple, les bases d'oxydation utilisables sont choisies parmi les para-phénylènediamines, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la para-phénylènediamine, la para-toluylènediamine, la 2 chloro para-phénylènediamine, la 2,3-diméthyl para-phénylènediamine, la 2,6 diméthyl para-phénylènediamine, la 2,6-diéthyl para-phénylènediamine, la 2,5 diméthyl para-phénylènediamine, la N,N-diméthyl para-phénylènediamine, la N,N-diéthyl para-phénylènediamine, la N,N-dipropyl para-phénylènediamine, la 4 amino N,N diéthyl 3-méthyl aniline, la N,N-bis-(β-hydroxyéthyl) para-phénylènediamine, la 4-N,N-bis-(β-hydroxyéthyl)amino-2-méthyl aniline, la 4 N,N bis (β hydroxyéthyl)amino-2-chloro aniline, la 2 ß hydroxyéthyl para-phénylènediamine, la 2 fluoro para-phénylènediamine, la 2 isopropyl para-phénylènediamine, la N (β hydroxypropyl) para-phénylènediamine, la 2 hydroxyméthyl para-phénylènediamine, la N,N diméthyl 3-méthyl para-phénylènediamine, la N,N (éthyl, ßhydroxyéthyl) para-phénylènediamine, la N-(ß,ß-dihydroxypropyl) para-phénylènediamine, la N (4' aminophényl) para-phénylènediamine, la N phényl para-phénylènediamine, la 2-ß-hydroxyéthyloxy para-phénylènediamine, la 2-ß-acétylaminoéthyloxy para-phénylènediamine, la N (ß-méthoxyéthyl) para-phénylène-diamine, la 4-aminophénylpyrrolidine, la 2-thiényl para-phénylènediamine, le 2-ß-hydroxyéthylamino-5-aminotoluène, la 3-hydroxy-1-(4'-aminophényl)pyrrolidine et leurs sels d'addition avec un acide.

Parmi les para-phénylènediamines citées ci-dessus, la para-phénylènediamine, la para-toluylènediamine, la 2 isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3 diméthyl para-phénylènediamine, la N,N bis-(β-hydroxyéthyl) para-phénylènediamine, la 2 chloro para-phénylènediamine, la 2-βacétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3 diamino propanol, la N,N'-bis-(β-hydroxyéthyl) N,N'-bis-(4'-aminophényl) éthylènediamine, la N,N' bis-(4-aminophényl) tétraméthylènediamine, la N,N' bis (β-hydroxyéthyl) N,N'-bis-(4-aminophényl) tétraméthylènediamine, la N,N' bis-(4-méthyl-aminophényl) tétraméthylènediamine, la N,N'-bis-(éthyl) N,N' bis (4'-amino, 3' méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino-3-méthyl phénol, le 4 amino-3 fluoro phénol, le 4 amino-3-hydroxyméthyl phénol, le 4-amino-2-méthyl phénol, le 4-amino-2 hydroxyméthyl phénol, le 4-amino-2-méthoxyméthyl phénol, le 4 amino-2 aminométhyl phénol, le 4-amino-2-(□-hydroxyéthyl aminométhyl)phénol, le 4 amino 2-fluoro phénol, et leurs sels d'addition avec un acide.

Parmi les ortho-aminophénols, on peut citer à titre d'exemple, le 2-aminophénol, le 2-amino-5-méthylphénol, le 2-amino-6 méthylphénol, le 5-acétamido-2 aminophénol, et leurs sels d'addition avec un acide.

Parmi les bases hétérocycliques, on peut citer à titre d'exemple, les dérivés pyridiniques, les dérivés pyrimidiniques, les dérivés pyrazoliques, les dérivés de pyrazolone, et leurs sels d'addition.

Parmi les dérivés pyridiniques, on peut citer les composés décrits par exemple dans les brevets GB 1 026 978 et GB 1 153 196, comme la 2,5 diamino pyridine, la 2-(4-méthoxyphényl)amino 3 amino pyridine, la 2,3 diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5 a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88 169571 ; JP 05 63124 ; EP 0770375 ou demande de brevet WO 96/15765 comme la 2,4,5,6-tétra-aminopyrimidine, la 4 hydroxy 2,5,6-triaminopyrimidine, la 2 hydroxy-4,5,6-triaminopyrimidine, la 2,4 dihydroxy-5,6-diaminopyrimidine, la 2,5,6 triaminopyrimidine, et les dérivés pyrazolo-pyrimidiniques tels ceux mentionnés dans la demande de brevet FR A 2750048 et parmi lesquels on peut citer la pyrazolo-[1,5-a]-pyrimidine-3,7-diamine ; la 2,5-diméthyl pyrazolo [1,5 a]-pyrimidine-3,7-diamine ; la pyrazolo-[1,5-a]-pyrimidine-3,5-diamine ; la 2,7 diméthyl pyrazolo-[1,5-a]-pyrimidine-3,5-diamine ; le 3-amino pyrazolo-[1,5 a]-pyrimidin-7-ol ; le 3-amino pyrazolo-[1,5-a]-pyrimidin-5-ol ; le 2 (3 amino pyrazolo-[1,5-a]-pyrimidin-7-ylamino)-éthanol, le 2 (7-amino pyrazolo-[1,5-a]-pyrimidin-3-ylamino)-éthanol, le 2-[(3-amino-pyrazolo[1,5-a]pyrimidin-7-yl)-(2 hydroxy-éthyl)-amino]-éthanol, le 2-[(7-amino-pyrazolo[1,5-a]pyrimidin-3-yl)-(2 hydroxy-éthyl)-amino]-éthanol, la 5,6-diméthyl pyrazolo-[1,5-a]-pyrimidine-3,7 diamine, la 2,6 diméthyl pyrazolo-[1,5-a]-pyrimidine-3,7-diamine, la 2, 5, N7, N7-tétraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques utilisables, on peut citer par exemple les composés décrits dans les brevets DE A 38 43 892, DE A 41 33 957 et demandes de brevet WO 94/08969, WO 94/08970, FR A-2 733 749 et DE A 195 43 988 comme le 4,5 diamino-1-méthylpyrazole, le 4,5-diamino-1-(β-hydroxyéthyl)¬pyrazole, le 3,4 diaminopyrazole, le 4,5-diamino-1 (4' chlorobenzyl)pyrazole, le 4,5-diamino-1,3-diméthyl-pyrazole, le 4,5 diamino-3-méthyl-1-phénylpyrazole, le 4,5-diamino-1-méthyl-3-phénylpyrazole, le 4-amino-1,3-diméthyl-5-hydrazinopyrazole, le 1 benzyl-4,5-diamino-3-méthylpyrazole, le 4,5-diamino-3-tert-butyl-1-méthylpyrazole, le 4,5-diamino-1-tert-butyl-3-méthylpyrazole, le 4,5-diamino-1 (β-hydroxyéthyl)-3-méthylpyrazole, le 4,5 diamino-1 éthyl-3-méthylpyrazole, le 4,5-diamino-1-éthyl-3 (4' méthoxyphényl)-pyrazole, le 4,5-diamino-1-éthyl-3 hydroxyméthylpyrazole, le 4,5-diamino-3-hydroxyméthyl-1 méthylpyrazole, le 4,5-diamino-3 hydroxyméthyl-1-isopropylpyrazole, le 4,5 diamino-3-méthyl-1 isopropylpyrazole, le 4-amino-5-(2'-aminoéthyl)amino-1,3 diméthylpyrazole, le 3,4,5 triaminopyrazole, le 1-méthyl-3,4,5-triaminopyrazole, le 3,5-diamino-1 méthyl-4-méthylaminopyrazole, le 3,5-diamino-4 (β-hydroxyéthyl)amino-1 méthylpyrazole, et leurs sels d'addition.

Parmi les dérivés de pyrazolone utilisables, on peut citer par exemple les composés suivants et leurs sels d'addition :

2,3-diaminodihydropyrazolone ;
4,5-diamino-1,2-diméthyl-1,2-dihydropyrazol-3-one ;
4-amino-5-méthylamino-1,2-diméthyl-1,2-dihydropyrazol-3-one ;
4-amino-5-diméthylamino-1 ,2-diméthyl-1 ,2-dihydropyrazol-3-one ;
4-amino-5-(2-hydroxyéthyl)amino-1 ,2-diméthyl-1 ,2-dihydropyrazol-3-one ;
4-amino-5-(pyrrolidin-1-yl)-1,2-diméthyl-1,2-dihydropyrazol-3-one ;
4-amino-5-(pipéridin-1-yl)-1,2-diméthyl-1,2-dihydropyrazol-3-one ;
4,5-diamino-1,2-di-(2-hydroxyéthyl)-1,2-dihydropyrazol-3-one ;
4-amino-5-méthylamino-1,2-di-(2-hydroxyéthyl)-1,2-dihydropyrazol-3-one ;
4-amino-5-diméthylamino-1 ,2-di-(2-hydroxyéthyl)-1 ,2-dihydropyrazol-3-one ;
4-amino-5-(2-hydroxyéthyl)amino-1,2-di-(2-hydroxyéthyl)-1,2-dihydropyrazol-3-one ;
4-amino-5-(pyrrolidin-1-yl)-1,2-di-(2-hydroxyéthyl)-1,2-dihydropyrazol-3-one ;
4-amino-5-(pipéridin-1-yl)-1,2-di-(2-hydroxyéthyl)-1,2-dihydropyrazol-3-one ;
4,5-diamino-1,2-diéthyl-1,2-dihydropyrazol-3-one ;
4,5-diamino-1,2-diphényl-1,2-dihydropyrazol-3-one ;
4,5-diamino-1-éthyl-2-méthyl-1,2-dihydropyrazol-3-one ;
4,5-diamino-2-éthyl-1-méthyl-1,2-dihydropyrazol-3-one ;
4,5-diamino-1-phényl-2-méthyl-1,2-dihydropyrazol-3-one ;
4,5-diamino-2-phényl-1-méthyl-1,2-dihydropyrazol-3-one ;
4,5-diamino-1-(2-hydroxyéthyl)-2-méthyl-1,2-dihydropyrazol-3-one ;
4,5-diamino-2-(2-hydroxyéthyl)-1-méthyl-1,2-dihydropyrazol-3-one ;
2,3-diamino-6,7-dihydro-1H,5H-pyrazolo[1,2-a]pyrazol-1-one ;
2-amino-3-méthylamino-6,7-dihydro-1 H,5H-pyrazolo[1,2-a]pyrazol-1-one;
2-amino-3-diméthylamino-6,7-dihydro-1 H,5H-pyrazolo[1,2-a]pyrazol-1-one ;
2-amino-3-éthylamino-6,7-dihydro-1 H,5H-pyrazolo[1,2-a]pyrazol-1-one ;
2-amino-3-isopropylamino-6,7-dihydro-1 H,5H-pyrazolo[1,2-a]pyrazol-1-one ;
2-amino-3-(2-hydroxyéthyl)amino-6,7-dihydro-1 H,5H-pyrazolo[1,2 a]pyrazol-1-one;
2-amino-3-(2-hydroxypropyl)amino-6,7-dihydro-1H,5H-pyrazolo[1,2-a]pyrazol-1-one;
2-amino-3-bis(2-hydroxyéthyl)amino-6,7-dihydro-1 H,5H-pyrazolo[1,2-a]pyrazol-1-one;
2-amino-3-(pyrrolidin-1-yl)-6,7-dihydro-1H,5H-pyrazolo[1,2-a]pyrazol-1-one ;
2-amino-3-(3-hydroxy-pyrrolidin-1-yl)-6,7-dihydro-1H,5H-pyrazolo[1,2-a]pyrazol-1-one ;

2-amino-3-(pipéridin-1-yl)-6,7-dihydro-1H,5H-pyrazolo[1,2-a]pyrazol-1-one ;
2,3-diamino-6-hydroxy-6,7-dihydro-1H,5H-pyrazolo[1,2-a]pyrazol-1-one ;
2,3-diamino-6-méthyl-6,7-dihydro-1 H,5H-pyrazolo[1,2-a]pyrazol-1-one ;
2,3-diamino-6,6-diméthyl-6,7-dihydro-1 H,5H-pyrazolo[1,2-a]pyrazol-1-one ;
2,3-diamino-5,6,7,8-tétrahydro-1 H,6H-pyridazino[1,2-a]pyrazol-1-one ;
2,3-diamino-5,8-dihydro-1H,6H-pyridazino[1,2-a]pyrazol-1-one ;
4-amino-5-diméthylamino-1 ,2-diéthyl-1 ,2-dihydropyrazol-3-one ;
4-amino-1,2-diéthyl-5-éthylamino-1,2-dihydropyrazol-3-one ;
4- amino -1 ,2-diéthyl-5-isopropylamino-1 ,2-dihydropyrazol-3-one ;
4- amino -1 ,2-diéthyl-5-(2-hydroxyéthylamino)-1 ,2-dihydropyrazol-3-one ;
4-amino-5-(2-diméthylaminoéthylamino)-1,2-diéthyl-1,2-dihydropyrazol-3-one ;
4-amino-5-[bis(2-hydroxyéthyl)amino]-1 ,2-diéthyl-1 ,2-dihydropyrazol-3-one ;
4-amino-1,2-diéthyl-5-(3-imidazol-1-yl-propylamino)-1,2-dihydropyrazol-3-one ;
4-amino-1.2-diéthyl-5-(3-hydroxypyrrolidin-1-yl)-1,2-dihydropyrazol-3-one ;
4-amino-5-pyrrolidin-1-yl-1,2-diéthyl-1,2-dihydropyrazol-3-one ;
4-amino-5-(3-diméthylaminopyrrolidin-1-yl)-1,2-diéthyl-1,2-dihydropyrazol-3-one ;
4-amino-1,2-diéthyl-5-(4-méthylpipérazin-1-yl)pyrazolidin-3-one.

La concentration en base(s) d'oxydation va en général de 0,001 à 20% en poids, de préférence de 0,005 à 10 % en poids, et encore plus préférentiellement de 0,01 à 5% en poids, par rapport au poids total de la composition.

Le (ou les) coupleur(s) d'oxydation présent(s) dans les compositions de l'invention, peut (ou peuvent) être choisi(s) parmi les coupleurs benzéniques, les coupleurs hétérocycliques, les coupleurs naphtaléniques, et leurs sels d'addition.

A titre de coupleurs benzéniques utilisables dans les compositions selon l'invention, on peut citer les méta-aminophénols, les méta-phénylènediamines, les méta-diphénols, ainsi que leurs sels d'addition.

Parmi les coupleurs préférés, on peut citer le 2-méthyl-5-aminophénol, le 5-N-(β-hydroxyéthyl)amino-2-méthyl phénol, le 6-chloro-2-méthyl-5-aminophénol, le 3-aminophénol, le 1,3-dihydroxy benzène, le 1,3-dihydroxy-2-méthyl benzène, le 4-chloro-1,3-dihydroxy benzène, le 2,4-diamino-1-(β-hydroxyéthyloxy)benzène, le 2-amino-4-(β-hydroxyéthylamino)-1-méthoxybenzène, le 1,3-diamino benzène, le 1,3-bis-(2,4-diaminophénoxy)propane, la 3-uréido aniline, le 3-uréido 1-diméthylamino benzène, le sésamol, le 1-ß-hydroxyéthylamino-3,4-méthylènedioxybenzène, l'ß-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(ß-hydroxyéthyl)amino-3,4-méthylène dioxybenzène, le 2,6-bis-(β-hydroxyéthylamino)toluène et leurs sels d'addition avec un acide.

La concentration en coupleur(s) d'oxydation va en général de 0,001 à 20 % en poids, de préférence de 0,005 à 10% en poids, et encore plus préférentiellement de 0,01 à 5 % en poids, par rapport au poids total de la composition.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le ou les alcools gras utiles dans la composition de la présente invention sont des alcools gras à chaine longue comprenant au moins 20 atomes de carbone, de préférence entre 20 et 30 atomes de carbone.

Les alcools gras utiles dans la présente invention comportent une chaîne hydrocarbonée comprenant au moins 20 atomes de carbone qui peut être linéaire ou ramifiée, saturée ou insaturée. De préférence, ce sont des alcools gras primaires qui présentent une chaîne linéaire et saturée. On peut notamment citer à titre d'exemples l'alcool béhénylique, l'alcool arachidylique, l'alcool lignocérylique, l'alcool cérylique et l'alcool montanylique et leurs mélanges à chaîne longue comprenant au moins 20 atomes de carbone.

Comme mélange d'alcools gras particulièrement préféré dans la composition selon l'invention, on peut utiliser, par exemple, le mélange d'alcools gras constitué de 76% en poids d'alcool béhénylique, de 17% en poids d'alcool arachidylique, de 1,5% en poids d'alcool lignocérylique, de 5% en poids d'alcool stéarylique et de 0,5% en poids d'alcool cétylique. Ce mélange est vendu sous la dénomination Nafol® 1822 C par la société CONDEA. Comme autres exemples, on peut également citer le mélange vendu sous la dénomination Nafol® 2298 par la société CONDEA, qui comprend 98% en poids d'alcool béhénylique ; le mélange vendu sous la dénomination Nafol® 20-22 par la société CONDEA, qui comprend 30 % en poids d'alcool béhénylique, 58% en poids d'alcool arachidylique et 6 % en poids d'alcool lignocérylique ; ou encore le mélange vendu sous la dénomination Nafol® 20+ par la société CONDEA, qui comprend 50% en poids d'alcool arachidylique, 29% en poids d'alcool béhénylique, 14% en poids d'alcool lignocérylique et 6% en poids d'alcool stéarylique.

La quantité du mélange d'alcools gras à chaine longue comprenant au moins 20 atomes de carbone dans la

composition selon l'invention est notamment comprise entre 0,1 et 20% en poids, de préférence entre 0,2 et 10%, mieux entre 0,5 et 6% en poids par rapport au poids total de la composition.

La composition tinctoriale conforme à l'invention peut, en outre, contenir un ou plusieurs colorant(s) direct(s) pouvant notamment être choisi(s) parmi les colorants nitrés benzéniques, les colorants directs azoïques, les colorants directs méthiniques, les colorants anthraquinoniques, les colorants xanthéniques, les colorants triarylméthaniques et leurs sels d'addition. Ces colorants directs peuvent être de nature non ionique, anionique ou cationique.

Le milieu utilisé dans les compositions selon la présente invention est un milieu aqueux ou un milieu contenant de l'eau et au moins un solvant organique.

Le (ou les) solvant(s) organique(s) utilisé(s) dans les compositions selon la présente invention peut (ou peuvent) être choisi(s) parmi les alcools monohydroxylés et les polyols différents de ceux utiles dans la composition de l'invention.

A titre d'alcools monohydroxylés utilisables, on peut citer les alcools inférieurs en $C_1$-$C_4$ comme l'éthanol, l'isopropanol, le tertiobutanol, le n-butanol, et leurs mélanges. De préférence, l'alcool utilisé est l'éthanol.

À titre de polyols utilisables, on peut citer le propylèneglycol, les polyéthylèneglycols, la glycérine. A titre de solvants organiques, on peut aussi citer les éthers de polyols comme le 2-butoxyéthanol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

La concentration en solvant(s) organique(s) dans les compositions selon la présente invention est comprise de préférence entre 0 et 30%, et de manière plus préférée entre 0 et 20% en poids par rapport au poids total de la composition.

Les compositions selon la présente demande peuvent également contenir, un ou plusieurs agent(s) épaississant(s) encore appelé "agent(s) d'ajustement de la rhéologie" différents des dérivés non-ioniques de cellulose à substituant(s) hydrophobe(s) de l'invention.

L'agent (ou les agents) d'ajustement de la rhéologie peut (ou peuvent) être choisi(s) parmi les agents épaississants minéraux ou organiques, et en particulier les épaississants associatifs polymériques, les alcools gras différents de ceux de la présente invention tels que l'alcool oléïque, les dérivés cellulosiques autres que les dérivés de cellulose non-ioniques à substituant(s) hydrophobe(s) (A) selon l'invention (hydroxyéthylcellulose, hydroxypropylcellulose, carboxyméthylcellulose) et les gommes d'origine microbienne (gomme de xanthane, gomme de scléroglucane).

La concentration en agent(s) épaississant(s) est comprise de préférence entre 0,01 et 20% en poids, et de manière plus préférée entre 1 et 10% en poids, par rapport au poids total de la composition

La composition tinctoriale conforme à l'invention peut également contenir un ou plusieurs adjuvant(s) utilisé(s) classiquement dans les compositions pour la teinture des cheveux.

Par «adjuvant(s)», on entend un (ou des) additif(s), différent(s) des composés précités, tels que les agents tensioactifs anioniques, cationiques, non-ioniques, amphotères, zwittérioniques ou leurs mélanges ; les polymères non-ioniques, amphotères, zwittérioniques, anioniques, cationiques autres que les dérivés de cellulose non-ioniques à substituant(s) hydrophobe(s) (A) selon l'invention, ou leurs mélanges ; les agents de pénétration ; les agents séquestrants ; les parfums ; les tampons ; les agents dispersants ; les agents conditionneurs tels que par exemple des silicones volatiles ou non volatiles, modifiées ou non modifiées ; les agents filmogènes ; les céramides ; les agents conservateurs ; les agents opacifiants ; les vitamines ; les acides aminés ; les oligopeptides ; les peptides ; les protéines hydrolysées ou non, modifiées ou non ; les enzymes ; les acides et alcools gras ramifiés ou non ; les cires animales, végétales ou minérales ; les acides organiques hydroxylés ; les alcools gras autres que ceux de l'invention ; les filtres UV ; les agents anti-oxydants et les agents anti-radicaux libres ; les agents antipelliculaires ; les agents régulateurs de séborrhée ; les agents apaisants ; les huiles minérales, végétales ou animales ; les polyisobutènes et poly($\alpha$-oléfines) ; les pigments ; les acides, bases, plastifiants, charges minérales, nacres, paillettes ; les agents anti-statiques et les agents réducteurs.

Le (ou les) adjuvant(s) ci-dessus est (ou sont), en général, présent(s) en quantité comprise, pour chacun d'eux, de préférence entre 0,01 et 40 % en poids, et de manière plus préférée entre 0,1 et 25 % en poids par rapport au poids de la composition.

Bien entendu, l'homme de l'art veillera à choisir ce (ou ces) éventuel(s) composé(s) complémentaire(s) de manière telle que les propriétés avantageuses attachées intrinsèquement à la composition de teinture d'oxydation conforme à l'invention ne soient pas, ou substantiellement pas, altérées par la (ou les) adjonction(s) envisagée(s).

Le pH de la composition tinctoriale conforme à l'invention va généralement de 3 à 12 environ, et de préférence de 5 à 11 environ. Il peut être ajusté à la valeur désirée au moyen d'agent(s) acidifiant(s) ou alcalinisant(s), habituellement utilisés en teinture des fibres kératiniques ou bien encore à l'aide de système(s) tampon(s) classique(s).

Parmi les agents acidifiants, on peut citer, à titre d'exemple, les acides minéraux ou organiques comme l'acide chlorhydrique, l'acide orthophosphorique, l'acide sulfurique, les acides sulfoniques et les acides carboxyliques comme l'acide acétique, l'acide tartrique, l'acide citrique et l'acide lactique.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante :

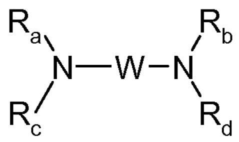

dans laquelle :

- W est un reste propylène éventuellement substitué par un groupe hydroxyle ou un groupe alkyle en $C_1$-$C_4$ ;
- $R_a$, $R_b$, $R_c$ et $R_d$, identiques ou différents, représentent un atome d'hydrogène, un groupe alkyle en $C_1$-$C_4$ ou hydroxyalkyle en $C_1$-$C_4$.

La composition tinctoriale selon l'invention peut se présenter sous des formes diverses, telles que sous forme de crèmes, de gels, ou sous toute autre forme appropriée pour réaliser une teinture des fibres kératiniques, et notamment des cheveux humains.

Le procédé de teinture des fibres kératiniques de la présente invention est un procédé dans lequel on applique sur les fibres la composition selon la présente invention telle que définie précédemment, de préférence en présence d'au moins un agent oxydant, pendant un temps suffisant pour développer la couleur désirée. La couleur peut être révélée à pH acide, neutre ou alcalin et l'agent (ou les agents) oxydant(s) peut (ou peuvent) être ajouté(s) à la composition de l'invention juste au moment de l'emploi ou il(s) peut (ou peuvent) être mis en oeuvre à partir d'une composition oxydante le(s) contenant, appliquée simultanément ou séquentiellement à la composition de l'invention.

Selon un mode de réalisation particulier, la composition selon la présente invention est une composition prête à l'emploi, mélangée, de préférence au moment de l'emploi, à une composition contenant, dans un milieu approprié pour la teinture, au moins un agent oxydant, cet agent (ou ces agents) oxydant(s) étant présent(s) en une quantité suffisante pour développer une coloration. Le mélange obtenu est ensuite appliqué sur les fibres kératiniques. Après un temps de pause de 3 à 50 minutes environ, de préférence, 5 à 30 minutes environ, les fibres kératiniques sont rincées, lavées au shampooing, rincées à nouveau, puis séchées.

Les agents oxydants classiquement utilisés pour la teinture d'oxydation des fibres kératiniques sont, par exemple, le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, ces oxydoréductases étant éventuellement associées à leurs cofacteurs habituels tels que l'acide urique pour les uricases. L'agent oxydant préféré est le peroxyde d'hydrogène.

La composition oxydante peut également renfermer divers adjuvants utilisés classiquement dans les compositions pour la teinture des cheveux, tels que définis précédemment.

Le pH de la composition oxydante renfermant l'agent oxydant est tel qu'après mélange avec la composition tinctoriale, le pH de la composition résultante appliquée sur les fibres kératiniques varie, de préférence, de 3 à 12 environ, et préférentiellement, de 5 à 10. Il peut être ajusté à la valeur désirée au moyen d'agent(s) acidifiant(s) ou alcalinisant(s) habituellement utilisé(s) en teinture des fibres kératiniques, tel(s) que défini(s) précédemment.

La composition prête à l'emploi qui est finalement appliquée sur les fibres kératiniques, peut se présenter sous des formes diverses, telles que sous forme de crèmes, de gels ou sous toute autre forme appropriée pour réaliser une teinture des fibres kératiniques, et notamment des fibres kératiniques humaines tels que les cheveux.

L'invention a aussi pour objet un dispositif à plusieurs compartiments ou "kit" de teinture comprenant au moins un premier compartiment contenant la composition tinctoriale définie ci dessus et au moins un deuxième compartiment contenant une composition oxydante. Ce dispositif peut être équipé d'un moyen permettant de délivrer sur les cheveux le mélange souhaité, tel que les dispositifs décrits dans la demande de brevet FR A 2 586 913.

Les exemples qui suivent servent à illustrer l'invention sans toutefois présenter un caractère limitatif.

Exemples

**<u>Composition 1</u>**

Les compositions suivantes ont été réalisées, les quantités indiquées sont en gramme sauf indication différente.

| | |
|---|---|
| RESORCINOL | 0,685 |
| Monoéthalonamide d'acide stéarique | 4,8 |

(suite)

| | |
|---|---|
| Acide oléique | 3 |
| CETYL HYDROXYETHYLCELLULOSE (Natrosol plus grade 330CS commercialisé par Hercules | 0,4 |
| Ammoniaque à 20% | 5 |
| DIOXIDE de Titane | 0,3 |
| ETHANOLAMINE | 0,8 |
| OLETH-10 | 1,8 |
| Solution aqueuse à 40% en poids de Polyquaternium-6 (chlorure de polydiméthyldiallylammonium (MERQUAT 100, commercialisé par ONDEO/NALCO | 1,6 |
| m-AMINOPHENOL | 0,14 |
| EDTA | 0,2 |
| 2,4-DIAMINOPHENOXYETHANOL HCl | 0,02 |
| Solution aqueuse à 60 % en poids de chlorure d'hexadiméthrine (Mexomère PO commercialisé par la société Chimex) | 1,2 |
| réducteur | qs |
| antioxydant | qs |
| HYDROXYPROPYL METHYLCELLULOSE | 0,191 |
| OLETH-30 | 1,5 |
| STEARETH-2 | 5,5 |
| Alcools en C20-C22 (Nafol 2022 EN commercialisé par la société Sasol) | 3 |
| TOLUENE-2,5-DIAMINE | 0,7623 |
| Eau | Qs 100 |

**Protocole d'application**

Chaque composition est mélangée extemporanément, avec une fois et demie son poids avec une composition oxydante de pH voisin de 3 (eau oxygénée à 20 volumes) (6 % en poids d'$H_2O_2$). Le mélange se fait facilement et présente une bonne viscosité ; il est appliqué facilement sur des cheveux gris, à 90 % de cheveux blancs, à raison de 10 g pour 1 g de cheveux, pendant 30 minutes. Les cheveux sont ensuite rincés, lavés avec un shampooing standard et séchés.

La coloration capillaire est évaluée de manière visuelle. On obtient ainsi une coloration des cheveux dans une nuance châtain à reflet rouge acajou.

Ces colorations possèdent de bonnes propriétés notamment en termes de sélectivité et d'intensité. Les compositions obtenues sont stables dans le temps.

**Composition 2**

Les compositions suivantes ont été réalisées, les quantités indiquées sont en gramme sauf indication différente.

| | |
|---|---|
| Monoéthalonamide d'acide stéarique | 4,8 |
| ACIDE OLEIQUE | 3 |
| CETYL HYDROXYETHYLCELLULOSE( polysurf 67 commercialisé par Hercules) | 0.5 |
| p-AMI NOPHENOL | 0,24 |
| AMMONIAQUE à 20% | 5 |
| TITANIUMDIOXIDE | 0,3 |

(suite)

| | |
|---|---|
| ETHANOLAMI NE | 0,8 |
| 2-AMI NO-3-HYDROXYPYRI DI NE | 0,2 |
| 6-HYDROXYI NDOLE | 0,01 |
| Solution aqueuse à 40% en poids de polyquaternium-6 (chlorure de polydiméthyldiallylammonium , MERQUAT 100, commercialisé par ONDEO/NALCO | 1,6 |
| 4-AMINO-2-HYDROXYTOLUENE | 0,24 |
| 2-METHYL-5-HYDROXYETHYLAMI NOPHENOL | 0,15 |
| m-AMINOPHENOL | 0,024 |
| EDTA | 0,2 |
| 2,4-DIAMINOPHENOXYETHANOL HCl | 0,02 |
| Solution aqueuse à 60 % en poids de chlorure d'hexadiméthrine (Mexomère PO commercialisé par la société Chimex) | 1,2 |
| réducteur | qs |
| antioxydant | qs |
| HYDROXYPROPYL METHYLCELLULOSE | 0,191 |
| OLETH-30 | 1,5 |
| PEG-40 STEARATE | 1,8 |
| STEARETH-2 | 5,5 |
| GLYCERYL LAURYL ETHER | 0,5 |
| Alcools en C20-C22 (Nafol 2022 EN commercialisé par la société Sasol) | 3 |
| TOLUENE-2,5-DIAMINE | 0,2772 |
| Eau | Qs 100 |

L'application sur les cheveux est effectuée selon le protocole de l'exemple 1. On obtient ainsi une coloration des cheveux dans une nuance blond à reflet acajou cuivré.

<u>Les compositions suivantes ont été préparées (quantité exprimée en g %):</u>

| | Composition A | Composition B (Invention) | Composition C |
|---|---|---|---|
| AMMONIUM HYDROXIDE | 10 | 10 | 10 |
| ERYTHORBIC ACID | 0,50 | 0,50 | 0,50 |
| ETHANOLAMINE | 0,70 | 0,70 | 0,70 |
| EDTA | 0,20 | 0,20 | 0,20 |
| SODIUM SULFITE | 0,50 | 0,50 | 0,50 |
| TITANIUM DIOXIDE | 0,30 | 0,30 | 0,30 |
| 4-AMINO-2-HYDROXYTOLUENE | 0,2460 | 0,2460 | 0,2460 |
| p-PHENYLENEDIAMINE | 0,2160 | 0,2160 | 0,2160 |
| Alcool stearylique | 2,64 (*) | | |

(suite)

| | Composition A | Composition B (Invention) | Composition C |
|---|---|---|---|
| Mélange d'alcools linéaires C18/C20/C22/C24 7/58/30/6 (Nom comercial Nafol 2022) | | 3 (*) | 3 |
| CETYL HYDROXYETHYLCELLULOSE | 0,40 | 0,40 | 1,40 |
| Acide oleique | 3 | 3 | 3 |
| STEARETH-2 | 5,50 | 5,50 | 5,50 |
| STEARAMIDE MEA (96) (and) ETHANOLAMINE (2) (and) STEARIC ACID (2) ( | 5 | 5 | 5 |
| OLETH-30 | 1,50 | 1,50 | 1,50 |
| eau | Qs 100 | Qs 100 | Qs 100 |

Préparation de la composition A comparative

3g% de Nafol 2022 ayant les proportions suivantes C18/C20/C22/C24 7/58/30/6 représentent
0,21 g% de C18
1,74 g% de C20 = 0,0058 mole de $CH_3(CH_2)_{19}OH$
0,9 g% de C22 = 0,0027 mole de $CH_3(CH_2)_{21}OH$
0,18 g% de C24= 0,0005 mole de $CH_3(CH_{2)23}OH$

Donc le mélange de Nafol contient 0,0090 mole d'alcool en C20, C22, C24. Dans la composition A cette quantité d'alcool gras à chaine longue a été remplacé par 0,0090 mole d'alcool en C18 (alcool stearylique) soit 2,43 g.
Pour être comparable en quantité d'alcool en C18, les 3g% de Nafol 2022 ont été remplavé dans la composition A par 2,64 g% d'alcool stéarylique ( 0,21 + 2,43 ).
Au moment de l'emploi, chaque composition est mélangée avec 1 fois ½ son poids d'oxydant à 20 volumes (6% H2O2).
Chaque mélange est ensuite appliqué sur des mèches de cheveux à 90% de cheveux blancs naturels (BN) et permanentés (BP), à raison de 15g de mélange par g de cheveux.
Après 30 mn de pause à température ambiante, les mèches sont rincées, lavées avec un shampooing standard et séchées.

Mesures colorimétriques

La coloration des mèches est évaluée par des mesures colorimétriques dans le système CIE Lab à l'aide du spectrocolorimètre_Konica Minolta CM-2600d
Dans ce système, L représente l'intensité, plus la valeur de L* est faible, plus la coloration obtenues es intense La chromaticité est mesurée par les valeurs a* et b*, a* représentant l'axe ruge/vert et b* l'axe jaune/bleu

**SELECTIVITE DE LA COLORATION**

La sélectivité de la coloration est la variation de la couleur entre des cheveux naturels et des cheveux permanentés Les cheveux naturels sont représentatif de la nature des cheveux à la racine alors que les cheveux permanentés sont représentatifs de la nature des cheveux à la pointe.
La sélectivité est mesurée par :

ΔE, qui est la variation de la couleur entre les cheveux naturels et les cheveux permanents, est obtenu à partir de la formule :

$$\Delta E = \sqrt{(L^* - L_o^*)^2 + (a^* - a_o^*)^2 + (b^* - b_o^*)^2}$$

dans laquelle L* représente l'intensité a* et b*, la chromaticité des cheveux colorés naturels et L0* représente l'intensité et a0* et b0* la chromaticité des cheveux colorés permanenté. Plus la valeur de ΔE est faible, plus la sélectivité est faible et la coloration uniforme le long des cheveux.

Les résultats sont reportés dans le tableau ci-dessous

| | Type cheveux | L* | A* | B* | ΔE |
|---|---|---|---|---|---|
| A | BN | 26,97 | 15,26 | -0,68 | 5,86 |
| | BP | 21,29 | 15,06 | 0,76 | |
| B (inv) | BN | 23,99 | 16,38 | 1 | 1,72 |
| | BP | 23,01 | 16,1 | -0,39 | |
| C | BN | 26,50 | 16,48 | 0,33 | 5,70 |
| | BP | 20,91 | 15,48 | -0,16 | |

Ces résultats montrent que la composition de l'invention permet d'obtenir une puissance de la coloration plus importante sur cheveux naturels. Par ailleurs, ces résultats montrent une amélioration de la sélectivité avec la composition de l'invention ce qui est représentatif d'une meilleure homogénéité de la coloration de la racine à la pointe des cheveux.

## Revendications

**1.** Composition tinctoriale pour fibres kératiniques, comprenant, dans un milieu approprié pour la teinture :

(A) un ou plusieurs dérivé(s) non ionique(s) de cellulose comprenant un ou plusieurs substituant(s) hydrophobe(s) comprenant de 8 à 30 atomes de carbone en quantité comprise entre 0,1 et 1 % en poids par rapport au poids total de la composition ,
(B) un ou plusieurs colorants d'oxydation, et
(C) un ou plusieurs alcools gras comprenant au moins 20 atomes de carbone.

**2.** Composition tinctoriale selon la revendication 1, **caractérisée en ce que** le dérivé non ionique de cellulose (A) est une hydroxyéthylcellulose substituée par un ou plusieurs substituant(s) hydrophobe(s) comprenant de 8 à 30 atomes de carbone.

**3.** Composition tinctoriale selon l'une quelconque des revendications précédentes, **caractérisée en ce que** le substituant hydrophobe est un groupe alkyle en $C_{10}$-$C_{22}$.

**4.** Composition tinctoriale selon l'une quelconque des revendications précédentes, **caractérisée en ce que** le substituant hydrophobe est un groupement cétyle.

**5.** Composition tinctoriale selon l'une quelconque des revendications précédentes, **caractérisée en ce que** le degré de substitution hydrophobe va de 0,1 à 10% en poids, de préférence de 0,1 à 1% en poids et de manière plus préférée de 0,4 à 0,8% en poids, du poids total du polymère.

**6.** Composition tinctoriale selon l'une quelconque des revendications précédentes, **caractérisée en ce que** le colorant d'oxydation (C) est choisi parmi les bases d'oxydation, les coupleurs d'oxydation, et leurs sels d'addition.

**7.** Composition tinctoriale selon la revendication précédente, **caractérisée en ce que** le colorant d'oxydation est une base d'oxydation choisie parmi les bases d'oxydation para-phénylènediamines, les bis-phénylalkylènediamines, les para-aminophénols, les bis-para-aminophénols, les ortho-aminophénols, les bases hétérocycliques et leurs sels d'addition.

**8.** Composition tinctoriale selon l'une quelconque des revendications précédentes **caractérisée en ce que** le colorant d'oxydation est un coupleur d'oxydation choisi parmi les coupleurs benzéniques, les coupleurs hétérocycliques, les coupleurs naphtaléniques, et leurs sels d'addition.

**9.** Composition tinctoriale selon la revendication précédente, **caractérisée en ce que** le coupleur benzénique est choisi parmi les méta-aminophénols, les méta-phénylènediamines, les méta-diphénols, et leurs sels d'addition.

**10.** Composition selon l'une quelconque des revendications précédentes dans laquelle l'alcool gras comprend de 20 à 24 atomes de carbone.

**11.** Composition selon l'une quelconque des revendications précédentes dans laquelle le ou les alcools gras comprenant au moins 20 atomes de carbone représentent de 0,1 à 20 et de préférence de 0,2 à 10% du poids total de la composition.

**12.** Composition tinctoriale selon l'une quelconque des revendications précédentes, **caractérisée en ce qu'**elle comprend un ou plusieurs colorant(s) direct(s) choisi(s) parmi les colorants nitrés benzéniques, les colorants directs azoïques, les colorants directs méthiniques, les colorants anthraquinoniques, les colorants xanthéniques, les colorants triarylméthaniques et leurs sels d'addition.

**13.** Composition tinctoriale selon l'une quelconque des revendications précédentes, **caractérisée en ce qu'**elle comprend au moins un agent oxydant, de préférence le peroxyde d'hydrogène.

**14.** Procédé de teinture d'oxydation des fibres kératiniques, **caractérisé en ce qu'**on applique sur les fibres une composition tinctoriale telle que définie dans l'une quelconque des revendications 1 à 12 en présence d'au moins un agent oxydant, pendant un temps suffisant pour développer la couleur désirée.

**15.** Dispositif à plusieurs compartiments, **caractérisé en ce qu'**il comprend au moins un premier compartiment contenant une composition tinctoriale telle que définie dans l'une quelconque des revendications 1 à 12 et au moins un deuxième compartiment contenant au moins un agent oxydant.

## Patentansprüche

**1.** Färbezusammensetzung für Keratinfasern, die in einem für die Färbung geeigneten Medium Folgendes umfasst:

(A) ein oder mehrere nichtionische Cellulosederivate mit einem oder mehreren hydrophoben Substituenten mit 8 bis 30 Kohlenstoffatomen in einer Menge zwischen 0,1 und 1 Gew.-%, bezogen auf das Gesamtgewicht der Zusammensetzung,
(B) einen oder mehrere Oxidationsfarbstoffe und
(C) einen oder mehrere Fettalkohole mit mindestens 20 Kohlenstoffatomen.

**2.** Färbezusammensetzung nach Anspruch 1, **dadurch gekennzeichnet, dass** es sich bei dem nichtionischen Cellulosederivat (A) um eine durch einen oder mehrere hydrophobe Substituenten mit 8 bis 30 Kohlenstoffatomen substituierte Hydroxyethylcellulose handelt.

**3.** Färbezusammensetzung nach einem der vorhergehenden Ansprüche, **dadurch gekennzeichnet, dass** es sich bei dem hydrophoben Substituenten um eine $C_{10}$-$C_{22}$-Alkylgruppe handelt.

**4.** Färbezusammensetzung nach einem der vorhergehenden Ansprüche, **dadurch gekennzeichnet, dass** es sich bei dem hydrophoben Substituenten um eine Cetylgruppe handelt.

**5.** Färbezusammensetzung nach einem der vorhergehenden Ansprüche, **dadurch gekennzeichnet, dass** der Grad der hydrophoben Substitution 0,1 bis 10 Gew.-%, vorzugsweise 0,1 bis 1 Gew.-% und weiter bevorzugt 0,4 bis 0,8 Gew.-%, bezogen auf das Gesamtgewicht des Polymers, beträgt.

**6.** Färbezusammensetzung nach einem der vorhergehenden Ansprüche, **dadurch gekennzeichnet, dass** der Oxidationsfarbstoff (B) aus Oxidationsbasen, Oxidationskupplern und Additionssalzen davon ausgewählt ist.

7. Färbezusammensetzung nach dem vorhergehenden Anspruch, **dadurch gekennzeichnet, dass** es sich bei dem Oxidationsfarbstoff um eine Oxidationsbase, die aus para-Phenylendiamin-Oxidationsbasen, Bisphenylalkylendiaminen, para-Aminophenolen, Bis-para-aminophenolen, ortho-Aminophenolen, heterocyclischen Basen und Additionssalzen davon ausgewählt ist, handelt.

8. Färbezusammensetzung nach einem der vorhergehenden Ansprüche, **dadurch gekennzeichnet, dass** es sich bei dem Oxidationsfarbstoff um einen Oxidationskuppler, der aus Benzol-Kupplern, heterocyclischen Kupplern, Naphthalin-Kupplern und Additionssalzen davon ausgewählt ist, handelt.

9. Färbezusammensetzung nach dem vorhergehenden Anspruch, **dadurch gekennzeichnet, dass** der Benzol-Kuppler aus meta-Aminophenolen, meta-Phenylendiaminen, meta-Diphenolen und Additionssalzen davon ausgewählt ist.

10. Zusammensetzung nach einem der vorhergehenden Ansprüche, wobei der Fettalkohol 20 bis 24 Kohlenstoffatome umfasst.

11. Zusammensetzung nach einem der vorhergehenden Ansprüche, wobei der bzw. die Fettalkohole mit mindestens 20 Kahlenstoffatamen 0,1 bis 20% und vorzugsweise 0,2 bis 10% des Gesamtgewichts der Zusammensetzung ausmachen.

12. Färbezusammensetzung nach einem der vorhergehenden Ansprüche, **dadurch gekennzeichnet, dass** sie einen oder mehrere Direktfarbstoffe, die aus Nitrobenzol-Farbstoffen, Azo-Direktfarbstoffen, Methin-Direktfarbstoffen, Anthrachinon-Farbstoffen, Xanthen-Farbstoffen, Triarylmethan-Farbstoffen und Additionssalzen davon ausgewählt sind, umfasst.

13. Färbezusammensetzung nach einem der vorhergehenden Ansprüche, **dadurch gekennzeichnet, dass** sie mindestens ein Oxidationsmittel, vorzugsweise Wasserstoffperoxid, umfasst.

14. Verfahren zum Oxidationsfärben von Keratinfasern, **dadurch gekennzeichnet, dass** man auf die Fasern eine Färbezusammensetzung gemäß einem der Ansprüche 1 bis 12 in Gegenwart mindestens eines Oxidationsmittels über einen zur Entwicklung der gewünschten Färbung ausreichenden Zeitraum aufbringt.

15. Vorrichtung mit mehreren Kompartimenten, **dadurch gekennzeichnet, dass** sie mindestens ein erstes Compartiment, das eine Färbezusammensetzung gemäß einem der Ansprüche 1 bis 12 enthält, und mindestens ein zweites Kompartiment, das mindestens ein Oxidationsmittel enthält, umfasst.

## Claims

1. Dye composition for keratin fibres, comprising, in a medium suitable for dyeing:

   (A) one or more nonionic derivative(s) of cellulose comprising one or more hydrophobic substituent(s) containing from 8 to 30 carbon atoms in an amount of between 0.1% and 1% by weight, relative to the total weight of the composition,
   (B) one or more oxidation dyes, and
   (C) one or more fatty alcohols containing at least 20 carbon atoms.

2. Dye composition according to Claim 1, **characterized in that** the nonionic derivative of cellulose (A) is a hydroxyethylcellulose substituted with one or more hydrophobic substituent(s) containing from 8 to 30 carbon atoms.

3. Dye composition according to either one of the preceding claims, **characterized in that** the hydrophobic substituent is a $C_{10}$-$C_{22}$ alkyl group.

4. Dye composition according to any one of the preceding claims, **characterized in that** the hydrophobic substituent is a cetyl group.

5. Dye composition according to any one of the preceding claims, **characterized in that** the degree of hydrophobic substitution ranges from 0.1% to 10% by weight, preferably from 0.1% to 1% by weight, and more preferably from

0.4% to 0.8% by weight, of the total weight of the polymer.

6. Dye composition according to any one of the preceding claims, **characterized in that** the oxidation dye (B) is chosen from oxidation bases and oxidation couplers, and addition salts thereof.

7. Dye composition according to the preceding claim, **characterized in that** the oxidation dye is an oxidation base chosen from the oxidation bases: paraphenylenediamines, bisphenylalkylenediamines, para-aminophenols, les bis-para-aminophenols, ortho-aminophenols and heterocyclic bases, and addition salts thereof.

8. Dye composition according to any one of the preceding claims, **characterized in that** the oxidation dye is an oxidation coupler chosen from benzene couplers, heterocyclic couplers and naphthalene couplers, and addition salts thereof.

9. Dye composition according to the preceding claims, **characterized in that** the benzene coupler is chosen from meta-aminophenols, meta-phenylenediamines and meta-diphenols, and addition salts thereof.

10. Composition according to any one of the preceding claims in which the fatty alcohol contains from 20 to 24 carbon atoms.

11. Composition according to any one of the preceding claims in which the fatty alcohol(s) containing at least 20 carbon atoms represent(s) from 0.1% to 20%, and preferably from 0.2% to 10%, of the total weight of the composition.

12. Dye composition according to any one of the preceding claims, **characterized in that** it comprises one or more direct dye(s) chosen from nitrobenzene dyes, azo direct dyes, methine direct dyes, anthraquinone dyes, xanthene dyes and triarylmethane dyes, and addition salts thereof.

13. Dye composition according to any one of the preceding claims, **characterized in that** it comprises at least one oxidizing agent, preferably hydrogen peroxide.

14. Process for the oxidation dyeing of keratin fibres, **characterized in that** a dye composition as defined in any one of Claims 1 to 12 is applied to the fibres in the presence of at least one oxidizing agent for a period of time sufficient to develop the desired colour.

15. Multicompartment device, **characterized in that** it comprises at least a first compartment containing a dye composition as defined in any one of Claims 1 to 12 and at least a second compartment containing at least one oxidizing agent.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9803150 A
- EP 1707190 A
- FR 2803197 A
- GB 1026978 A
- GB 1153196 A
- FR 2801308
- DE 2359399
- JP 63169571 A
- JP 5063124 A
- EP 0770375 A
- WO 9615765 A
- FR 2750048 A
- DE 3843892 A
- DE 4133957 A
- WO 9408969 A
- WO 9408970 A
- FR 2733749 A
- DE 19543988 A
- FR 2586913 A